Europäisches Patentamt

European Patent Office

Office européen des brevets

(19)

(11) Veröffentlichungsnummer: **0 016 947**
B1

(12) **EUROPÄISCHE PATENTSCHRIFT**

(45) Veröffentlichungstag der Patentschrift: **13.07.83**

(21) Anmeldenummer: **80100841.8**

(22) Anmeldetag: **20.02.80**

(51) Int. Cl.³: **C 12 Q 1/00, C 12 Q 1/54, C 12 Q 1/60, C 12 Q 1/62**

(54) Verfahren und Reagenz zur enzymatischen Bestimmung von Enzymsubstraten.

(30) Priorität: **04.04.79 DE 2913553**

(43) Veröffentlichungstag der Anmeldung:
**15.10.80 Patentblatt 80/21**

(45) Bekanntmachung des Hinweises auf die Patenterteilung:
**13.07.83 Patentblatt 83/28**

(84) Benannte Vertragsstaaten:
**AT BE CH DE FR GB IT LU NL SE**

(56) Entgegenhaltungen:
**BE - A - 856 461**
**DE - A - 2 417 230**
**FR - A - 1 589 037**
**FR - A - 2 185 289**
**GB - A - 1 385 320**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH**
**Sandhofer Strasse 112-132 Postfach 31 01 20**
**D-6800 Mannheim 31-Waldhof (DE)**

(72) Erfinder: **Scheibe, Peter, Dr.**
**Schremelstrasse 39**
**D-8000 München 60 (DE)**
Erfinder: **Bernt, Erich**
**Grünwalder Strasse 34**
**D-8000 München 90 (DE)**
Erfinder: **Klose, Sigmar, Dr.**
**Maxhöhe 17**
**D-8131 Berg (DE)**

(74) Vertreter: **Weickmann, Heinrich, Dipl.-Ing. et al,**
**Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska**
**Möhlstrasse 22**
**D-8000 München 86 (DE)**

(56) Entgegenhaltungen:
**CHEMICAL ABSTRACTS, Band 89, Nr. 17, 23,
Oktober 1978, Zusammenfassung 142782t,
Seite 268, Columbus, Ohio, US, H. G.
EISENWIENER: "Assumptions on the
production of reliable clinical-chemical analysis
results. Part 2. Blank values and their significant
possibilities"**

**0 016 947**

## Verfahren und Reagenz zur enzymatischen Bestimmung von Enzymsubstraten

Die Erfindung betrifft ein Verfahren und ein Reagenz zur enzymatischen Bestimmung von Enzymsubstraten.

Enzymatische Bestimmungen von Enzymsubstraten, wie z.B. Glucose, Harnsäure, Cholesterin, werden häufig dadurch gestört, daß in den Reagenzien enthaltene Substanzen, beispielsweise reduzierende oder oxidierende Substanzen, mit Zwischenprodukten oder Endprodukten des Testsystems reagieren. Diese störende Nebenreaktion kann rein chemisch ablaufen oder die Störung kann durch Hemmung des Enzyms, Eingang in die enzymatische Reaktion als konkurrierendes Substrat und dergleichen erfolgen. In jedem Fall wird die Konzentration des eigentlichen Indikatorprodukts, welches gemessen wird, bei quantitativen, insbensondere photometrischen Bestimmungen, also beispielsweise des gebildeten Farbstoffs, vermindert. Das für die Richtigkeit des Verfahrens essentielle proportionale Verhältnis zwischen nachzuweisender Substratkonzentration und Konzentration des gemessenen Indikatorprodukts wird auf diese Weise beseitigt. Die Wiederfindung des nachzuweisenden Substrats weicht damit von 100% ab. Mit anderen Worten werden falsche Ergebnisse erhalten.

In der DE—A 24 17 230 wird ein Verfahren zur enzymatischen Bestimmung der Konzentration von Substraten durch Messung der die jeweilige Substratkonzentration kennzeichnenden Reaktionsgeschwindigkeit offenbart. Die dabei durch Einmischen der Enzyme in den Reaktionsansatz verursachte Störung ermöglicht eine korrekte Messung erste einige Sekunden nach dem Einmischen.

Der Erfindung liegt die Aufgabe zugrunde, dieses Problem zu beseitigen und ein Verfahren und ein Reagenz für die enzymatische Bestimmung eines Enzymsubstrats in Gegenwart von Störsubstanzen zu schaffen, welches zu richtigen Ergebnissen führt und die oben aufgeführten Schwierigkeiten beseitigt.

Gelöst wird diese Aufgabe erfindungsgemäß durch ein Verfahren zur enzymatischen Bestimmung eines Enzymsubstrats in Gegenwart von Störsubstanzen, die mit dem Substrat selbst oder mit einem Zwischen- oder Endprodukt der Indikatorreaktion reagieren können, welches dadurch gekennzeichnet ist, daß man vor Zugabe der zu untersuchenden Probe zuerst eine bestimmte Menge des Substrats mit dem Reagenz zur Umsetzung bringt, bis es abreagiert hat, und erst danach die zu bestimmende Probe zusetzt.

Durch den Zusatz der bestimmten Substratmenge, die stets deutlich unter der Substratbestimmungskapazität des Analysensystems liegt, wird der Ablauf einer Vorreaktion veranlaßt, bis alles zugesetzte Substrat verbraucht ist. Im Rahmen dieser Vorreaktion werden auch die störenden Substanzen vollständig verbraucht. Sobald diese Reaktion zum Stillstand gekommen ist, wird dann die eigentliche zu untersuchende Probe mit einem unbekannten Gehalt an dem zu bestimmenden Substrat zugesetzt. Die Menge des zu bestimmenden Substrats ergibt sich dann aus der Differenz zwischen dem Endwert der Vorreaktion für das zu messende Reaktionsprodukt und dem Endwert, der nach Zusatz der zu untersuchenden Probe erhalten wird. Wahlweise ist es auch möglich, die eigentliche Probenbestimmung kinetisch zu verfolgen, d.h. nicht Endwerte, sondern nur Reaktionsgeschwindigkeiten zu messen.

Für die Menge des erfindungsgemäß zuzusetzenden Substrats lassen sich keine allgemeinen Angaben machen. Es hängt diese Menge von der Art des Bestimmungssystems und der Menge der zu erwartenden Störsubstanzen ab. Im allgemeinen wird man einen genügenden Überschuß an Substrat bezüglich der Störsubstanzen anwenden, um mit Sicherheit anzuschließen, daß noch nicht abreagierte Störsubstanzen vorliegen, wenn die eigentliche unbekannte Probe zugesetzt wird. Andererseits wird man auch nicht zu viel Substrat zusetzen, um nicht für die eigentliche Probenreaktion einen zu hohen und die Genauigkeit der Meßreaktion negativ beeinflussenden Nullwert zu erhalten. Letzteres spieltz z.B. eine Rolle bei photometrischen Messungen eines als Verfahrensprodukt gebildeten Farbstoffes. Bei solchen, auf der photometrischen Bestimmung eines als Reaktionsprodukt auftretenden Farbstoffs beruhenden Methoden wird man die zugesetzte bestimmte Substratmenge so niedrig halten, daß noch keine störenden Konzentrationen an Farbstoff gebildet werden. Dies läßt sich in der Praxis in jedem Falle leicht erreichen, da ja ein derartiger Farbstoff oder ein sonstiges Indikatorprodukt erst von derjenigen Substratmenge gebildet wird, welche die zur Abreaktion der Störsubstanzen erforderliche Substratmenge übersteigt.

Beim Verfahren der Erfindung ist es nicht erforderlich, die Art der störenden Fremdsubstanzen zu kennen. Häufig handelt es sich hierbei zwar um reduzierende Substanzen, aber auch oxidierende oder sonstige störende Substanzen werden nach dem erfindungsgemäßen Verfahren mit Sicherheit ausgeschaltet.

Ein weiterer Gegenstand der Erfindung ist ein Reagenz zur enzymatischen Bestimmung eines Enzymsubstrats, enthaltend ein System zur Bestimmung des Substrats, welches aus wenigstens einem Enzym, wenigstens einer Puffersubstanz und wenigstens einer Indikatorsubstanz sowie gegebenenfalls Hilfsstoffen besteht und welches gekennzeichnet ist durch einen Gehalt an einer bestimmten Menge des Substrats im Unterschuß bezüglich der Substratbestimmungskapazität des Systems zur Bestimmung dieses Substrats.

Wenn das in Trockenform vorliegende, erfindungsgemäße Reagenz in Wasser aufgelöst wird, läuft die Reaktion zur Beseitigung der Störsubstanzen während und nach dem Lösungsvorgang bereits ab. Die so erhaltene Lösung kann dann direkt für die eigentliche Bestimmung des Enzymsubstrats eingesetzt oder aber erneut in ein Trockenreagens überführt werden, welches zur späteren Wiederauflösung vor Durchführung der Bestimmungsreaktion vorgesehen ist. Es ist aber auch möglich, das erfindungsgemäße Reagenz erste unmittelbar vor seiner Verwendung durch den Substratzusatz zu komplettieren oder alternativ eine andere, zum Start der Reaktion erforderliche Substanz erst nachträglich zuzusetzen, beispielsweise das Enzym oder im Fall einer mehrstufigen Reaktion mit mehreren enzymatischen Schritten, eines der für die Reaktion erforderlichen Enzyme.

Gemäß einer bevorzugten Ausführungsform des erfindungsgemäßen Reagenz enthält dieses Glucose als Substrat und das System zur Bestimmung des Substrats Glucose besteht aus Glucoseoxidase (GOD), Peroxidase (POD), Puffer und ABTS® (2,2'-Azino-di-[3-äthylbenzthiazolin-sulfonat(6)] als Farbreagenz.

Bei einem derartigen Reagenz werden, bezogen auf 100 ml wäßriger Lösung des Substratbestimmungssystems, etwa 750 bis 1500 U, vorzugsweise 900 bis 1200 U GOD einer spezifischen Aktivität von etwa 100 U/mg eingesetzt.

Die entsprechenden Mengen für POD einer Aktivität von ebenfalls ca. 100 U/mg betragen 10 bis 150 U, vorzugsweise 80 bis 120 U/100 ml gebrauchsfertiger Reagenzlösung. Die entsprechenden Konzentrationen für ABTS betragen 75 bis 150 mg, vorzugsweise 90 bis 120 mg/100 ml. Als Puffer kommt ein solcher von pH 6,5 bis 7,5, vorzugsweise 6,8 bis 7,2 in Betracht. Die Konzentration liegt zweckmäßig zwischen 50 und 200 mMol, vorzugsweise 80 bis 120 mMol/l. Als Puffer wird Natriumphosphatpuffer ($Na_2HPO_4/NaH_2PO_4$) bevorzugt. Beispiele für andere geeignete Puffer sind Phosphat-Tris-Puffer, Zitronensäure-Tris-Puffer, Weinsäure-Tris-Puffer und Maleinsäure-Tris-Puffer. Für die Konzentration und den pH-Wert gilt das gleiche wie oben ausgeführt.

Bei einem in der vorstehend beschriebenen Weise zusammengesetzten Reagenz beträgt der Glucosegehalt zweckmäßig 0,01 bis 0,05 mg, vorzugsweise 0,02 bis 0,04 mg/100 ml Lösung.

Bei einem weiteren bevorzugten erfindungsgemäßen Reagenz zur Glucosebestimmung enthält das System zur Bestimmung des Substrats neben GOD und POD als Farbbildungskomponenten 4-Aminophenazon (PAP) und Phenol. In diesem Fall werden an GOD pro 100 ml gebrauchsfertige Reagenslösung 3000 bis 5000 U, vorzugsweise 3800 bis 4200 U/100 ml, an POD 50 bis 240 U, vorzugsweise 160 bis 200 U/100 ml Lösung sowie 10 bis 20 mg, vorzugsweise 13 bis 17 mg/100 ml PAP und 120 bis 240 mg, vorzugsweise 160 bis 200 mg/100 ml Phenol eingesetzt. Als Puffer wird in diesem Fall Kaliumphosphat-Puffer des oben angegebenen pH-Wertbereichs bevorzugt. Die Konzentration sollte zweckmäßig zwischen 150 und 250 mMol, vorzugsweise 180 bis 220 mMol/l liegen. Bei diesem Reagenz beträgt der Glucosegehält zweckmäßig 0,05 bis 0,25 mg, vorzugsweise 0,1 bis 0,2 mg/100 ml Lösung.

Die obigen Angaben beziehen sich auf das gelöste Reagenz. Sie gelten in gleicher Weise auf die zur Herstellung von 100 ml Lösung bestimmte Menge Trockenreagens.

Ein weiteres bevorzugtes Reagenz enthält als Substrat Harnsäure und das System zur Bestimmung dieses Substrats besteht aus Uricase, POD, Farbbildner und Puffer. Als Farbbildner wird ein Gemisch von 2,4-Dichlorphenolat und 4-Aminoantipyrin bevorzugt.

Ein derartiges Reagenz enthält in einer zweckmäßigen Ausführungsform 35 bis 45 U, vorzugsweise 38 bis 42 U/100 ml gebrauchsfertiger Lösung an Uricase mit eine spezifischen Aktivität von etwa 9 U/mg. Die POD-Menge für ein Präparat von ca. 100 U/mg beträgt zweckmäßig 30 bis 40 U, vorzugsweise 33 bis 37 U/100 ml Lösung. Das 2,4-Dichlorphenolat, welches als Alkalisalz, Ammoniumsalz oder Aminosalz, vorzugsweise als Äthylammoniumsalz, eingesetzt wird, liegt zweckmäßig in Mengen zwischen 150 und 200 mg, vorzugsweise 165 bis 180 mg/100 ml, bezogen auf Äthylammoniumsalz, vor. Diese Mengenangaben verändern sich natürlich mit der Natur des Kations. Das 4-Aminoantipyrin wird zweckmäßig in 150 bis 250 mg, vorzugsweise 180 bis 220 mg/100 ml Reagenz eingesetzt.

Die Puffersubstanz soll im pH-Bereich 8,5 bis 9,5, vorzugsweise 8,7 bis 9,3, puffern. Die Konzentration beträgt zweckmäßig 100 bis 200 mMol, vorzugsweise 130 bis 180 mMol/l. Bevorzugt wird Tris-Citrat-Puffer, andere, gut geeignete Puffersubstanzen sind Tris-Weinsäure und Tris-Meleinsäure.

Bei diesem Reagenz liegt der Harnsäurezusatz zweckmäßig zwischen 0,05 und 0,25 mg, vorzugsweise 0,1 bis 0,2 mg/100 ml, bezogen auf das Lithium- oder Natriumsalz.

Noch ein weiteres bevorzugtes Reagenz gemäß der Erfindung enthält Cholesterin als Substrat und das System zur bestimmung dieses Substrats besteht aus Cholesterinesterase, Cholesterinoxidase, POD und Farbbildner sowie Puffersubstanz. Als Farbbildner eignet sich besonders eine Mischung von 4-Aminoantipyrin und Phenol.

In einer zweckmäßigen Ausführungsform enthält dieses Reagenz 15 bis 25 U, vorzugsweise 17 bis 23 U/100 ml Reagenzlösung einer Cholesterinesterase mit einer spezifischen Aktivität von ca. 20 U/mg, 20 bis 30 U, vorzugsweise 22 bis 28 U/100 ml einer Cholesterinoxidase einer spezifischen Aktivität von ca. 25 U/mg, 50 bis 200 U, vorzugsweise 130 bis 180 U/100 ml Lösung einer POD von ca. 100 U/mg, 15 bis 25 mg, vorzugsweise 17 bis 23 mg/100 ml Reagenslösung 4-Aminoantipyrin und 40 bis 60 mg, vorzugsweise 45 bis 55 mg/100 ml Phenol. Als Puffer wird eine geeignete Puffersubstanz, pH 7 bis 8,5 vorzugsweise 7,8 bis 8,2 mit einer Konzentration zwischen 150 und 250 mMol, vorzugsweise 180 bis 220 mMol/l eingesetzt. Besonders geeignet ist Kaliumphosphat-Puffer, jedoch eignen sich andere Puffersubstanzen, die im angegebenen Bereich zu puffern vermögen, ebenfalls. Der

4

Cholesteringehalt eines derartigen Reagens beträgt zweckmäßig 0,20 bis 0,60 mg, vorzugsweise 0,30 bis 0,50 mg/100 ml Reagenzlösung bzw. der zur Herstellung einer solchen Reagenzlösungsmenge vorgesehenen Trockenreagensmenge.

Es versteht sich, daß die für die oben beschriebenen bevorzugten erfindungsgemäßen Reagenzzusammensetzungen die angegebenen Enzymmengen nur für Enzyme der jeweils aufgeführten spezifischen Aktivität gelten und durch Präparate anderer Aktivität ersetzt werden können.

Es ist ersichtlich, daß erfindungsgemäß Störreaktionen bei der enzymatischen Bestimmung von Enzymsubstraten beseitigt werden können. Dies ermöglicht es, weniger reine Reagenzien einzusetzen, die Lagerfähigkeit von Reagenzien, bei denen Störsubstanzen während der Lagerung erst entstehen, zu verlängern und die Genauigkeit der Bestimmungen zu erhöhen.

Die folgenden Beispiele erläutern die Erfindung weiter.

Beispiel 1
Reagenz zur Glucosebestimmung nach der ABTS-Methode.

| | |
|---|---|
| Natriumphosphat-Puffer, pH 7.0 | 100 mMol/l |
| Glucoseoxidase (ca. 100 U/mg) | 1000 U/100 ml |
| Peroxidase (ca. 100 U/mg) | 80 U/100 ml |
| ABTS[+] | 100 mg/100 ml |
| Glucose | 0,05 mg/100 ml |

[+] 2,2'-Azino-di-[3-äthylbenzthiazolin-sulfonsäure(6)]-diammoniumsalz

Beispiel 2
Reagenz zur Glucosebestimmung nach der GOD-PAP-Methode

| | |
|---|---|
| Kaliumphosphat-Puffer, pH 7,1 | 205 mMol/l |
| Glucoseoxidase (GOD; ca. 100 U/mg) | 4100 U/100 ml |
| Peroxidase (POD; ca. 100 U/mg) | 190 U/100 ml |
| PAP (4-Aminophenazon) | 17 mg/100 ml |
| Phenol | 200 mg/100 ml |
| Glucose | 0,2 mg/100 ml |

Beispiel 3
Reagenz zur Cholesterinbestimmung

| | |
|---|---|
| Kaliumphosphat-Puffer, pH 7,9 | 200 mMol/l |
| Cholesterinesterase (ca. 20 U/mg) | 22 U/100 ml |
| Cholesterinoxidase (ca. 25 U/mg) | 24 U/100 ml |
| Peroxidase (ca. 100 U/mg) | 150 U/100 ml |
| 4-Aminoantipyrin | 21 mg/100 ml |
| Phenol | 47 mg/100 ml |
| Cholesterin | 0,4 mg/100 ml |

Beispiel 4
Reagenz zur Harnsäurebestimmung

| | |
|---|---|
| Tris-Citrat-Puffer, pH 9,1 | 150 mMol/l |
| Uricase (ca. 9 U/mg) | 39 U/100 ml |
| Peroxidase (ca. 100 U/mg) | 36 U/100 ml |
| 2,4-Dichlorphenolat-äthylammoniumsalz | 170 mg/100 ml |
| 4-Aminoantipyrin | 190 mg/100 ml |
| Harnsäure (Li- oder Na-Salz) | 0,16 mg/100 ml |

Beispiel 5
Vergleichende Glucosebestimmung
Um die erfindungsgemäß verbesserte Genauigkeit der Glucosebestimmung zu zeigen, wurde wie folgt vorgegangen:

Zwei Reagenzlösungen A und B wurden durch Auflösen von Reagenz gemäß Beispiel 1 in der erforderlichen Menge Wasser hergestellt. Bei Lösung A wurde jedoch der Glucosezusatz weggelassen. Zur Bestimmung wurde eine Glucose-Standardreihe mit von 50 bis 400 mg/100 ml ansteigendem Glucosegehalt sowie Humanserum eingesetzt.

Bestimmungsansatz:

| | |
|---|---|
| Wellenlänge: | Hg 436 nm |
| Küvette: | 1 cm Schichtdicke |
| Inkubationstemperatur: | 20 bis 25°C |

**0 016 947**

Messung erfolgte gegen Leerwert in handelsüblichem Photometer. In die Küvetten wurden folgende Lösungen einpipettiert:

|  | Leerwert | Standard | Probe |
|---|---|---|---|
| dest. Wasser | 0,1 ml | — | — |
| verdünnte Standardlösung | — | 0,1 ml | — |
| enteiweißte Probe | — | — | 0,1 ml |
| Testreagens (A/B) | 5,0 ml | 5,0 ml | 5,0 ml |

mischen, bei 20 bis 25°C inkubieren. Nach 25 bis 50 Minuten Extinktion der Probe ($E_{Probe}$) und Extinktion des Standards ($E_{Standard}$) gegen Leerwert messen.

Berechnung der Glucosekonzentration in der Probe:

$$c = 100 \times \frac{E_{Probe}}{E_{Standard}} \, [mg/100 \, ml]$$

Meßergebnisse:

Glucose-Standard-Reihe (50 bis 400 mg/100 ml)

| Glucosekonzentration | Extinktion in Reagenz A | B |
|---|---|---|
| 50 mg/100 ml | — | 0,095 |
| 100 mg/100 ml | 0,060 | 0,170 |
| 200 mg/100 ml | 0,236 | 0,353 |
| 300 mg/100 ml | 0,400 | 0,533 |
| 400 mg/100 ml | 0,577 | 0,700 |

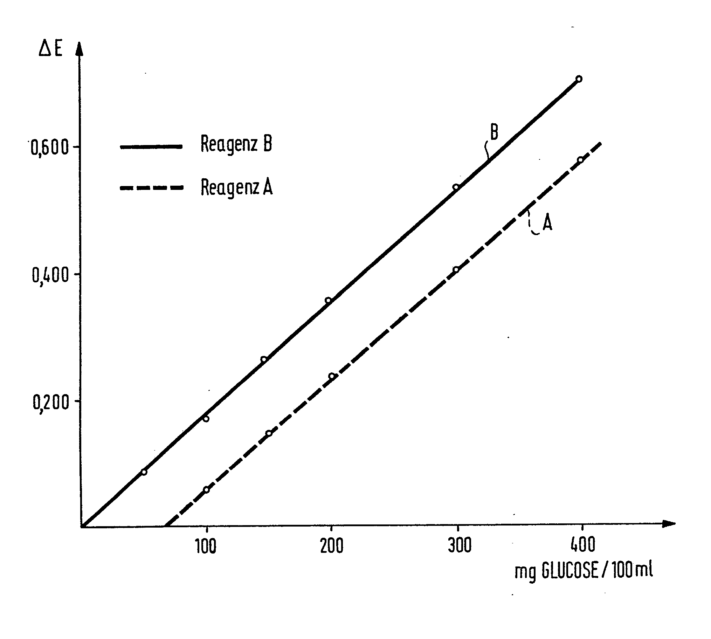

Die obigen Meßergebnisse sind in der Figur der beigefügten Zeichnung graphisch dargestellt. Die Kurve für Lösung A zeigt, daß Probeflüssigkeiten mit einem Glucosegehalt unter ca. 70 mg/100 ml nicht gemessen werden, da keine Extinktion, d.h. Farbentwicklung, auftritt. Bei Verwendung des allgemein üblichen Ein-Punkt-Standards (z.B. 100 mg Glucose/100 ml) werden Proben mit einer Glucosekonzentration zwischen 70 und 100 mg/100 ml zu niedrig, Proben mit Glucosegehalten über 100 mg/100 ml zu hoch gegenüber dem tatsächlichen Gehalt ermittelt. Nur Probekonzentrationen, die exakt der Konzentration des Standards entsprechen, liefern korrekte Meßwerte. Das erfindungsgemäße Reagenz B hingegen liefert durchweg richtige Werte.

Die Ergebnisse mit Humanserumproben wurden in der oben beschriebenen Weise durchgeführt, wobei zum Vergleich die nach der Hexokinase-Methode (Standardmethode) erhaltenen Werte bestimmt wurden. Die Ergebnisse zeigt nachstehende Tabelle.

|  | HK/G6P—DH (Standard-Methode) | Reagenz A | Reagenz B |
|---|---|---|---|
| Serum 1 | 53 mg/100 ml | kein Meßwert | 51 mg/100 ml |
| Serum 2 | 84 mg/100 ml | 55 mg/100 ml | 83,5 mg/100 ml |
| Serum 3 | 135 mg/100 ml | 213 mg/100 ml | 135 mg/100 ml |

**Patentansprüche**

1. Reagenz zur enzymatischen Bestimmung eines Enzymsubstrats, enthaltend ein System zur Bestimmung des Substrats, welches aus wenigstens einem Enzym, wenigstens einer Puffersubstanz und wenigstens einer Indikatorsubstanz sowie gegebenenfalls Hilfsstoffen besteht, gekennzeichnet durch einen Gehalt an einer bestimmten Menge des Substrats im Unterschuß bezüglich der Substratbestimmungskapazität des Systems zur Bestimmung dieses Substrats.

2. Reagenz nach Anspruch 1, dadurch gekennzeichnet, daß es Glucose als Substrat enthält und das System zur Bestimmung des Substrats aus Glucoseoxidase, Peroxidase, (2,2'-Azino-di-[3-äthylbenzthiazolin-sulfonat(6)] und Puffer besteht.

3. Reagenz nach Anspruch 1, dadurch gekennzeichnet, daß es Harnsäure als Substrat enthält und das System zur Bestimmung eines Substrats aus Uricase, Peroxidase, 2,4-Dichlorphenolat, 4-Aminoantipyrin und Puffer besteht.

4. Reagenz nach Anspruch 1, dadurch gekennzeichnet, daß es Cholesterin als Substrat enthält

6

und das System zur Bestimmung eines Substrats aus Cholesterinesterase, Cholesterinoxidase, Peroxidase, 4-Aminoantipyrin, Phenol und Puffer besteht.

5. Reagenz nach Anspruch 1, dadurch gekennzeichnet, daß es Glucose als Substrat enthält und das System zur Bestimmung eines Substrats aus Glucoseoxidase, Peroxidase, 4-Aminophenazon, Phenol und Puffer besteht.

6. Verfahren zur enzymatischen Bestimmung eines Enzymsubstrats in Gegenwart von Störsubstanzen, die mit dem Substrat selbst oder einem Zwischen- oder Endprodukt der Indikatorreaktion reagieren können, dadurch gekennzeichnet, daß man vor Zugabe der zu untersuchenden Probe zuerst eine bestimmte Menge des Substrats mit dem Reagenz zur Umsetzung bringt, bis es abreagiert hat und erst danach die zu bestimmende Probe zusetzt.

## Claims

1. Reagent for the enzymatic determination of an enzyme substrate, containing a system for the determination of the substrate, which consists of at least one enzyme, at least one buffer substance and at least one indicator substance, as well as possibly adjuvants, characterised by a content of a definite amount of the substrate in insufficient amount with regard to the substrate determination capacity of the system for the determination of this substrate.

2. Reagent according to claim 1, characterised in that it contains glucose as substrate and the system for the determination of the substrate consists of glucose oxidase, peroxidase, 2,2'-azino-di-(3-ethylbenzthiazoline-6-sulphonate) and buffer.

3. Reagent according to claim 1, characterised in that it contains uric acid as substrate and the system for the determination of a substrate consists of uricase, peroxidase, 2,4-dichlorophenolate, 4-aminoantipyrine and buffer.

4. Reagent according to claim 1, characterised in that it contains cholesterol as substrate and the system for the determination of a substrate consists of cholesterol esterase, cholesterol oxidase, peroxidase, 4-aminoantipyrine, phenol and buffer.

5. Reagent according to claim 1, characterised in that it contains glucose as substrate and the system for the determination of a substrate consists of glucose oxidase, peroxidase, 4-aminophenazone, phenol and buffer.

6. Process for the enzymatic determination of an enzyme substrate in the presence of disturbing substances which can react with the substrate itself or an intermediate or end product of the indicator reaction, characterised in that, before the addition of the sample to be investigated, a definite amount of the substrate is brought to reaction with the reagent until it has reacted completely and only thereafter is the sample to be determined added thereto.

## Revendications

1. Réactif pour la détermination enzymatique d'un substrat enzymatique, contenant un système pour la détermination du substrat, qui se compose d'au moins une enzyme, d'au moins une substance tampon et d'au moins une substance indicatrice, ainsi que le cas échéant d'adjuvants, caractérisé en ce qu'il contient une quantité déterminée du substrat en quantité insuffisante par rapport à la capacité de détermination du substrat du système pour la détermination de ce substrat.

2. Réactif suivant la revendication 1, caractérisé en ce qu'il contient du glucose comme substrat et en ce que le système pour la détermination du substrat se compose de glucose-oxydase, de peroxydase, de 2,2'-azino-di-[3-éthylbenzothiazoline-sulfonate(6)] et de tampon.

3. Réactif suivant la revendication 1, caractérisé en ce qu'il contient de l'acide urique comme substrat et en ce que le système pour la détermination d'un substrat se compose d'uricase, de peroxydase, de 2,4-dichlorophénolate, de 4-aminoantipyrine et de tampon.

4. Réactif suivant la revendication 1, caractérisé en ce qu'il contient du cholestérol comme substrat et en ce que le système pour la détermination d'un substrat se compose de cholestérolestérase, de cholestéroloxydase, de peroxydase, de 4-aminoantipyrine, de phénol et de tampon.

5. Réactif suivant la revendication 1, caractérisé en ce qu'il contient du glucose comme substrat et en ce que le système pour la détermination d'un substrat se compose de glucose-oxydase, de peroxydase, de 4-aminophénazone, de phénol et de tampon.

6. Procédé pour la détermination enzymatique d'un substrat enzymatique en présence de substances gênantes qui peuvent réagir avec le substrat lui-même ou avec un produit intermédiaire ou un produit final de la réaction de l'indicateur, caractérisé en ce qu'avant l'addition de l'échantillon à étudier, on fait d'abord réagir une quantité déterminée du substrat avec le réactif, jusqu'à ce qu'elle ait complètement réagi, et on ajoute seulement ensuite l'échantillon à déterminer.

0 016 947

1